(19) Europäisches Patentamt
European Patent Office
Office européen des brevets

(11) Publication number: **0 023 780**
**B1**

(12) **EUROPEAN PATENT SPECIFICATION**

(45) Date of publication of patent specification: **11.04.84**

(51) Int. Cl.³: **G 03 C 1/485,** G 03 C 1/06, G 03 C 5/26

(21) Application number: **80302365.4**

(22) Date of filing: **11.07.80**

(54) **Silver halide emulsions containing a nucleating agent, photographic elements, film unit and processes for the production of direct positive images.**

(30) Priority: **11.07.79 US 56588**
**19.02.80 US 122151**

(43) Date of publication of application:
**11.02.81 Bulletin 81/6**

(45) Publication of the grant of the patent:
**11.04.84 Bulletin 84/15**

(84) Designated Contracting States:
**DE FR GB**

(56) References cited:
**DE - A - 2 635 316**
**DE - A - 2 913 567**
**FR - A - 2 410 300**

(73) Proprietor: **EASTMAN KODAK COMPANY**
**343 State Street**
**Rochester New York 14650 (US)**

(72) Inventor: **Leone, Ronald Edmund**
**Kodak Park**
**Rochester, New York (US)**

(74) Representative: **Pepper, John Herbert et al,**
**KODAK LIMITED Patent Department P.O. Box 114**
**190 High Holborn**
**London WC1V 7EA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 023 780 B1

# Silver halide emulsions containing a nucleating agent, photographic elements, film unit and processes for the production of direct positive images

This invention relates to nucleated silver halide emulsions, photographic elements and processes for preparing direct positive images. More specifically, this invention relates to emulsions with silver halide grains capable of forming an internal latent image, the silver halide grains having a nucleating agent adsorbed to the surface thereof.

Photographic elements which produce images having an optical density directly related to the radiation received on exposure are said to be negative-working. A positive photographic image can be formed by producing a negative photographic image and then forming a second or positive photographic image. A direct-positive image is understood in photography to be a positive image that is formed without first forming a visible negative image. Positive dye images which are not direct-positive images are commonly produced in colour photography by reversal processing in which a negative silver image is formed and a complementary positive dye image is then formed in the same photographic element. The term "direct reversal" has been applied to direct-positive photographic elements and processing which produces a positive dye image without forming a negative silver image.

A conventional approach to forming direct-positive images is to use photographic elements employing internal latent image-forming silver halide grains. After imagewise exposure, the silver halide grains are developed with a surface developer. Such a developer leaves the latent image sites within the silver halide grains substantially unrevealed. Simultaneously, either by uniform light exposure or by the use of a nucleating agent, the silver halide grains are subjected to development conditions that would cause fogging of a negative-working photographic element. The internal latent image-forming silver halide grains which received actinic radiation during imagewise exposure developed under these conditions at a comparatively slow rate, as compared to the internal latent image-forming silver halide grains not imagewise exposed. The result is a direct-positive silver image. In colour photography, the oxidized developer that is produced during silver development may be used to produce a corresponding positive, direct reversal dye image. Multicolour direct reversal photographic images have been extensively investigated in connection with image-transfer photography.

It is advantageous to employ nucleating agents in preference to uniform light exposure in the direct-positive process described above. The term "nucleating agent" is employed herein in its art-recognized usage to mean a fogging agent capable of permitting the slective development of internal latent image-forming silver halide grains which have not been imagewise exposed in preference to the development of silver halide grains having an internal latent image formed by imagewise exposure.

A useful class of nucleating agents is arylhydrazides. These nucleating agents can be incorporated in a developer solution or directly within a photographic element. Significant advantages have been achieved by adsorbing certain arylhydrazide nucleating agents to the surface of internal latent image-forming silver halide grains. This permits small amounts of the nucleating agents to be employed, as compared with those which are non-adsorbed. However, this narrows the choice of arylhydrazide nucleating agents to those including an adsorption-promoting group.

A known class of adsorbed arylhydrazide nucleating agents is the acylhydrazinophenylthioureas described in U.S. Patent 4,030,925. These acylhydrazinophenylthioureas are characterized by the 3-position nirogen atom of the thiourea moiety being mono-substituted.

However, there is now a demand for nucleating agents having properties which are improved as compared with those disclosed in the prior art particularly as regards the image discrimination obtained in the processed element containing the nucleating agent. The foregoing improvement may now be achieved by the present invention which provides a silver halide emulsion comprising silver halide grains capable of forming an internal latent image, the silver halide grains having adsorbed on their surfaces an acylhydrazinophenylthiourea nucleating agent, characterized in that the nucleating agent has the formula

(I) 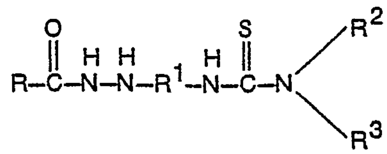

wherein

R is hydrogen, cycloalkyl which may be substituted with halogen, cyano or alkoxy groups or alkyl which may be substituted with halogen, alkoxy or phenyl groups;

$R^1$ is a phenylene group or a phenylene group substituted with alkyl, halogen or alkoxy; and

$R^2$ and $R^3$ are independently a) an alkyl, haloalkyl, alkoxyalkyl or phenylalkyl group having from 1 to 18 carbon atoms; b) a cycloalkyl group which may be substituted with halogen, cyano or alkoxy groups; c) a phenyl group having a positive Hammett sigma value-derived electron-withdrawing characteristic less than +0.50; or d) naphthyl; or

$R^2$ and $R^3$ together complete a heterocyclic group having a 5- or 6-membered ring which may have at least one additional hetero atom selected from nitrogen, oxygen, sulfur and selenium;

the alkyl groups, except as otherwise noted, in each instance have 1 to 6 carbon atoms; and the cycloalkyl groups have 3 to 10 carbon atoms.

Preferred 3,3-disubstituted acylhydrazinophenylthiourea nucleating agents used in the invention are those of the formula:

(II) 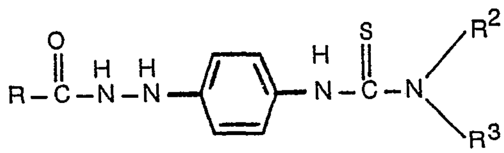

wherein

R is hydrogen or methyl and

$R^2$ and $R^3$ are independently an alkyl group having 1 to 6 carbon atoms, a phenylalkyl group wherein the alkyl has 1 to 6 carbon atoms, or a phenyl group having a positive Hammett sigma value-derived electron-withdrawing characteristic less positive than +0.50; or

$R^2$ and $R^3$ together complete a saturated heterocyclic nucleus having a 5- or 6-membered ring containing as additional hetero atom at least one of nitrogen, oxygen, sulfur and selenium.

The

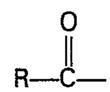

in formula (I) is an acyl group which is the residue of a carboxylic acid, such as one of the acylic carboxylic acids, including formic acid, acetic acid, propionic acid, butyric acid, higher homologues of these acids having up to 7 carbon atoms, and halogen, alkoxy, phenyl and equivalent substituted derivaties thereof. The acyl group is more generally formed by an unsubstituted acyclic aliphatic carboxylic acid having 1 to 5 carbon atoms. Preferred acyl groups are formyl and acetyl. As between compounds which differ solely in terms of having a formyl or an acetyl group, the compound containing the formyl group exhibits higher nucleating agent activity. The alkyl groups in the substituents to the carboxylic acids have 1 to 6 carbon atoms, preferably 1 to 4 carbon atoms.

In addition to the acyclic aliphatic carboxylic acids, it is recognized that the carboxylic acid can be chosen so that R is a cycloalkyl group having 3 to 10 carbon atoms, such as cyclopropyl, cyclobutyl, cyclopentyl, cyclohexyl, methylcyclohexyl, cyclooctyl, cyclodecyl and bridged ring variations, such as bornyl and isobornyl groups. Cyclohexyl is a preferred cycloalkyl group. The use of alkoxy-, cyano- and halogen-substituted cycloalkyl groups are suitable.

As indicated by $R^1$ in formula (I), preferred 3,3-disubstituted arylhydrazinophenylthioureas employed in the practice of this invention contain a phenylene or substituted phenylene group. Specifically preferred phenylene groups are *m*- and *p*-phenylene groups. Exemplary of preferred phenylene substituents are alkoxy substituents having 1 to 6 carbon atoms, alkyl substituents having 1 to 6 carbon atoms, fluoro-, chloro-, bromo- and iodo-substituents. Unsubstituted *p*-phenylene groups are preferred. Preferred alkyl groups are those which have 1 to 4 carbon atoms.

In formula (I) $R^2$ and $R^3$ can independently take a variety of forms. One specifically contemplated form can be an alkyl group, a haloalkyl group, alkoxyalkyl group or phenylalkyl group having a total of up to 18, preferably up to 12, carbon atoms. Specifically $R^2$ and/or $R^3$ can take the form of a methyl, ethyl, propyl, butyl, pentyl, hexyl, heptyl, octyl, nonyl, decyl or higher homologue group having up to 18 total carbon atoms; a fluoro-, chloro-, bromo-, iodo- methoxy-, ethoxy, propoxy-, butoxy- substituted alkyl groups wherein the total number of carbon atoms are of 2 to 18, and a phenyl-substituted alkyl group wherein the total number of carbon atoms is at least 7, as in the case of benzyl, and up to 18. In a preferred form indicated in formula (II) $R^2$ and/or $R^3$ can take the form of an alkyl or phenylalkyl group wherein the alkyl groups have in each instance from 1 to 6 carbon atoms.

In addition to the acyclic aliphatic and aromatic forms discussed above, $R^2$ and/or $R^3$ can take the form of a cycloalkyl group having 3 to 10 carbon atoms. Cyclopropyl, cyclobutyl, cyclopentyl, cyclohexyl, methylcyclohexyl, cyclooctyl, cyclodecyl and bridged ring variations, such as bornyl and isobornyl groups, can be used. Cyclohexyl is a preferred cycloalkyl. The use of alkoxy-, cyano- and halogen- substituted cycloalkyl groups is contemplated.

$R^2$ and/or $R^3$ can be a phenyl group having a positive Hammett sigma value-derived electron-withdrawing characteristics less than +0.50. $R^2$ and/or $R^3$ can be chosen from among phenyl groups having cyano, fluoro, chloro, bromo, iodo, alkyl having 1 to 6 carbon atoms and alkoxy groups having 1 to 6 carbon atoms as phenyl ring substituents. Phenyl ring substituents are preferred in the *para*- or 4-ring position.

Exemplary *meta*- and *para*-sigma values and procedures for their determination are set forth by J. Hine in *Physical Organic Chemistry*, second edition, page 87, published in 1962, by H. Van Bekkum,

P. E. Verkade and B. M. Wepster in *Rec. Trav. Chim.*, Volume 78, page 815, published in 1959, by P. R. Wells in *Chem. Revs.*, Volume 63, page 171, published in 1963, by H. H. Jaffe in *Chem. Revs.*, Volume 53, page 191, published in 1953, by M. J. S. Dewar and P. J. Grisdale in *J. Amer. Chem. Soc.*, Volume 84, page 3548, published in 1962, and by Barlin and Perrin in *Quart. Revs.*, Volume 20, page 75 *et seq*, published in 1966. For the purpose of this invention, *ortho-* substituents to the phenyl ring can be assigned to the published *para-* sigma values.

Rather than being independently chosen, $R^2$ and $R^3$ can together complete along with the 3-position nitrogen atom of the thiourea, a heterocyclic nucleus forming a 5- or 6-membered ring. The additional hetero ring atoms can be chosen from among nitrogen, oxygen, sulfur and selenium. The ring necessarily contains at least one hetero nitrogen atom. Exemplary rings include morpholino, piperidino, pyrrolidinyl, pyrrolinyl, thiomorpholino, thiazolidinyl, 4-thiazolinyl, selenazolidinyl, 4-selenazolinyl, imidazolidinyl, imidazolinyl, oxazolidinyl and 4-oxazolinyl rings. Preferred rings are saturated or otherwise constructed to avoid electron withdrawal from the 3-position nitrogen atom.

Preferred values from R and H, for $R^1$ phenyl, for each of $R^2$ and $R^3$, $CH_3$, benzyl and phenyl and for $R^2$ and $R^3$ together morpholino, piperidino and pyrrolidino.

To synthesize the 3,3-disubstituted acylhydrazinophenylthioureas used in the invention, nitrophenylhydrazine can be employed as a starting material and can be used to form the desired 1-acyl-2-(aminophenyl) hydrazine using procedures that are generally described in U.S. Patent 4,030,925.

A general procedure for synthesis using a 1-acyl-2-(aminophenyl)hydrazine as a starting material is to dissolve this material in acetone and cool the resulting solution to −78°C in a dry ice-acetone bath. The 1-acyl-2-(aminophenyl)hydrazine is then reacted with 1,1′-thiocarbonyldiimidazole. The reaction mixture is allowed to warm to 25°C and then the acetone solvent is removed. The remaining solid residue consists essentially of a mixture of a 2-acylhydrazinophenylisothiocyanate and imidazole. The solid is washed with water to remove the imidazole, and the remaining material is recrystallized from acetone to give the 2-acylhydrazinophenylisothiocyanate separated from the imidazole. The isothiocyanate is then reacted in a suitable solvent, such as ethanol or acetonitrile, with a secondary amine, where $R^2$ and $R^3$ are independent substituents, or a heterocyclic compound having a functionally equivalent nitrogen atom, where $R^2$ and $R^3$ together form a heterocyclic ring. After a few minutes reflux the 1-(2-acylhydrazinophenyl)-3,3-disubstituted thiourea separates from the solution as a precipitate. The solid product can be filtered off, washed with diethyl ether and dried.

This synthesis can be summarized as follows:

(III)

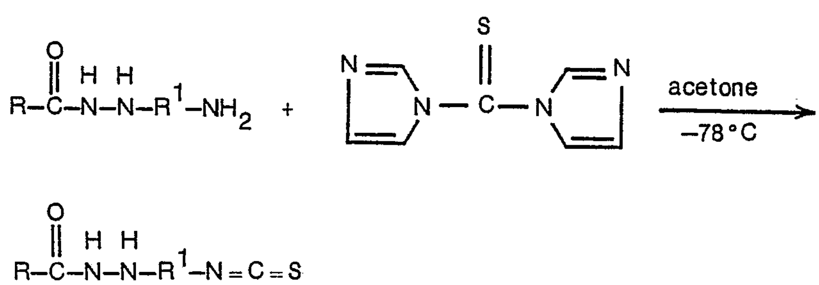


(IV)

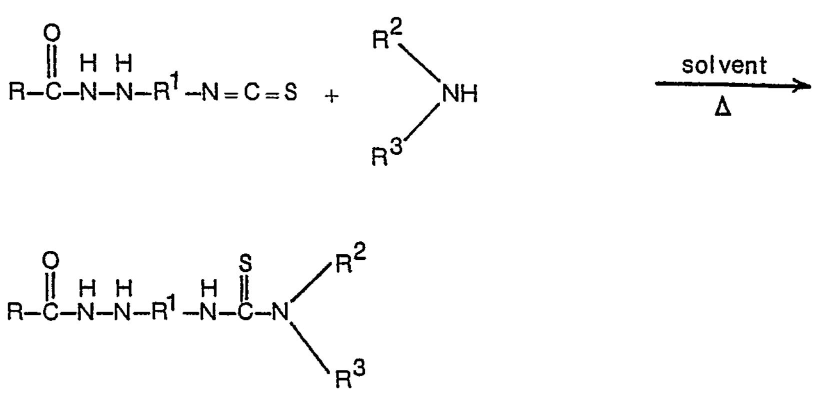


Where a thiocarbamoyl chloride is available which contains substituents corresponding to the desired $R^2$ and $R^3$ substituents, such as dimethyl or diethyl carbamoyl chloride, an alternative synthetic

route is to react the carbamoyl chloride with a desired 1-acyl-2-(aminophenyl)-hydrazine in an inert solvent, such as acetonitrile, in the presence of a base, such as triethylamine, under a nitrogen atmosphere at 25°C. The mixture is chilled and the product separates out of solution. The solid is filtered off and then stirred in warm water (e.g. 35°C) to remove hydrochloride salts. The remaining solid material represents the 3,3-disubstituted-1-(acylhydrazinophenyl)-thiourea.

This synthesis can be summarized as follows:

(IV)

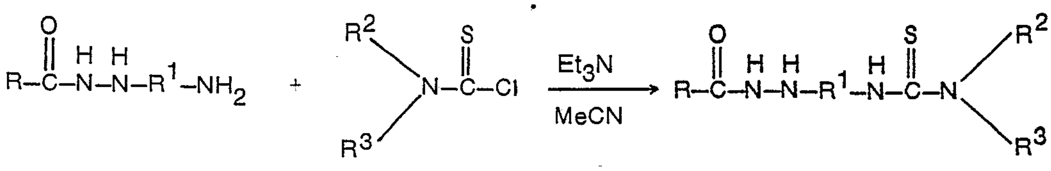


Illustrative specific 3,3-disubstituted acylhydrazinophenylthioureas useful in the practice of this invention include those set forth below in Table I.

TABLE I

| | |
|---|---|
| NA—1 | 1-[4-(2-formylhydrazino)phenyl]-3,3-dimethylthiourea |
| NA—2 | 1-[4-(2-formylhydrazino)phenyl]-3,3-di-(chloroethyl)thiourea |
| NA—3 | 1-[2-(2-formylhydrazino)phenyl]-3,3-dimethylthiourea |
| NA—4 | 1-[3-(2-formylhydrazino)phenyl]-3,3-dimethylthiourea |
| NA—5 | 1-[4-(2-acetylhydrazino)phenyl]-3,3-dimethylthiourea |
| NA—6 | 1-[4-(2-chloroacetylhydrazino)phenyl]-3,3-dimethylthiourea |
| NA—7 | 1-[4-(2-methoxyacetylhydrazino)phenyl]-3,3-dimethylthiourea |
| NA—8 | 1-[4-(2-heptanoylhydrazino)phenyl]-3,3-dimethylthiourea |
| NA—9 | 1-[4-(2-cyclobutanoylhydrazino)phenyl]-3,3-dimethylthiourea |
| NA—10 | 1-{4-(2-(4-chlorobutanoyl)hydrazino)-phenyl}-3,3-dimethylthiourea |
| NA—11 | 1-[4-(2-formylhydrazino)phenyl]-3,3-dibenzylthiourea |
| NA—12 | 1-[4-(2-acetylhydrazino)phenyl]-3,3-dibenzylthiourea |
| NA—13 | 1-{4-[2-(4-chlorobutanoyl)hydrazino]-phenyl}-3,3-dibenzylthiourea |
| NA—14 | 1-[4-(2-formylhydrazino)phenyl]-3-methyl-3-phenylthiourea |
| NA—15 | 1-[4-(2-formylhydrazino)phenyl]3,3-dibutylthiourea |
| NA—16 | 1-[4-(2-formylhydrazino)phenyl]-3-methyl-3-(2-ethoxy)ethylthiourea |
| NA—17 | 1-[4-(2-formylhydrazino)phenyl]-3,3-dihexylthiourea |
| NA—18 | 1-[4-(2-formylhydrazino)phenyl]-3,3-didodecylthiourea |
| NA—19 | 1-[4-(2-acetylhydrazino)phenyl]-3,3-dioctadecylthiourea |
| NA—20 | 1-[4-(2-bromoacetyl)phenyl]-3,3-dimethylthiourea |
| NA—21 | 4-[4-(2-formylhydrazino)phenylthiocarbamoyl]morpholine |
| NA—22 | 4-[4-(2-formylhydrazino)phenylthiocarbamoyl]thiomorpholine |

| | |
|---|---|
| NA—23 | 3-[4-(2-formylhydrazino)phenylthiocarbamoyl]thiazolidine |
| NA—24 | 3-[4-(2-formylhydrazino)phenylthiocarbamoyl]-4-thiazoline |
| NA—25 | 1-[4-(2-formylhydrazino)phenylthiocarbamoyl]-3-pyrroline |
| NA—26 | 1-[4-(2-formylhydrazino)phenylthiocarbamoyl]pyrrolidine |
| NA—27 | 1-[4-(2-formylhydrazino)phenylthiocarbamoyl]imidazolidine |
| NA—28 | 1-[4-(2-formylhydrazino)phenylthiocarbamoyl]-2-imidazoline |
| NA—29 | 3-[4-(2-formylhydrazino)phenylthiocarbamoyl]oxazolidine |
| NA—30 | 3-[4-(2-formylhydrazino)phenylthiocarbamoyl]-4-oxazoline |
| NA—31 | 1-[4-(2-formylhydrazino)phenyl]-3,3-(di-2-naphthyl)thiourea |
| NA—32 | 1-[4-(2-formylhydrazino)phenylthiocarbamoyl]piperidine |
| NA—33 | 1-[4-(2-chloroacetylhydrazino)phenylthiocarbamoyl]piperidine |

The 3,3-disubstituted arylhydrazinophenylthiourea nucleating agents can be employed with conventional photographic direct-positive silver halide emulsions containing silver halide grains capable of forming an internal latent image upon exposure to actinic radiation. As employed herein, the terms "internal latent image silver halide grains" and "silver halide grains capable of forming an internal latent image" are employed in the art-recognized sense of designating silver halide grains which produce substantially higher optical densities when coated, imagewise exposed and developed in an internal developer when comparably coated, exposed and developed in a surface developer. Preferred internal latent image silver halide grains are those which, when examined according to normal photographic testing techniques, by coating a test portion on a photographic support at a density of from 3 to 4 grams per square metre, exposing the a light intensity scale (such as, for example, with a 500-watt tungsten lamp at a distance of 61 cm) for a fixed time between 1 × $10^{-2}$ and 1 second and developing for 5 minutes at 25°C in 'Kodak' (registered trade mark) Developer DK—50 (a surface developer), provide a density of at least 0.5 less than when this testing procedure is repeated, substituting for the surface developer 'Kodak' Developer DK—50 containing 0.5 gram per litre of potassium iodide (and internal developer). The internal latent image silver halide grains most preferred for use in the practice of this invention are those which, when tested using an internal developer and a surface developer as indicated above, produce an optical density with the internal developer at least 5 times that produced by the surface developer. It is additionally preferred that the internal latent image silver halide grains produce an optical density of less than 0.4 and, most preferably, less than 0.25 when coated, exposed and developed in surface developer as indicated above that is, the silver halide grains are initially substantially unfogged and free of latent image on their surface.

The surface developer referred to herein as 'Kodak' Developer DK—50 is described in the *Handbook of Chemistry and Physics,* 30th edition, 1947, Chemical Rubber Publishing Company, Cleveland, Ohio, page 2558, and has the following composition:

| | |
|---|---|
| Water, (52°C) | 5.00.0 ml |
| N-methyl-p-aminophenol sulphate | 2.0 g |
| Sodium sulphite, desiccated | 30.0 g |
| Hydroquinone | 2.5 g |
| Sodium metaborate | 10.0 g |
| Potassium bromide | 0.5 g |
| Water to make | 1.0 litre |

Internal latent image silver halide grains which can be employed in the practice of this invention are well known in the art. Patents teaching the use of internal latent image silver halide grains in

photographic emulsions and elements include Davey et al U.S. Patent 2,592,250, Porter et al U.S. Patent 3,206,313, Milton U.S. Patent 3,761,266, Ridgway U.S. Patent 3,586,505; Gilman et al U.S. Patent 3,772,030; Gilman et al U.S. Patent 3,761,267 and Evans U.S. Patent 3,761,276.

The internal latent image silver halide grains preferably contain bromide as the predominant halide. The silver bromide grains can consist essentially of silver bromide or they can contain silver bromoiodide, silver chlorobromide, silver chlorobromoiodide or mixtures thereof. Internal latent image-forming sties can be incorporated into the grains by either physical or chemical internal sensitization. Davey et al, cited above, for example, teaches the physical formation of internal latent image-forming sites by the halide conversion technique. Chemical formation of internal latent image-forming sites can be produced through the use of sulphur, gold, selenium, tellurium and/or reduction sensitizers of the type described, for example, in Sheppard et al U.S. Patent 1,623,499; Waller et al U.S. Patent 2,399,083; McVeigh U.S. Patent 3,297,447 and Dunn U.S. Patent 3,297,446. Internal latent image sites can also be formed through the incorporation of metal dopants, particularly Group VIII noble metals, such as ruthenium, rhodium, palladium, iridium, osmium and platinum, as taught by Berriman U.S. Patent 3,367,778. The preferred foreign metal ions are polyvalent metal ions which include the above-noted Group VIII dopants, as well as polyvalent metal ions such as lead, antimony, bismuth, arsenic and the like. In highly preferred embodiments, the silver halide grains are formed in the presence of bismuth, lead or iridium ions. In a preferred approach, the internal latent image sites can be formed within the silver halide grains during precipitation of silver halide. In an alternate approach, a core grain can be formed which is treated to form the internal image sites and then a shell deposited over the core grains.

The silver halide grains employed in the practice of this invention are preferably monodispersed, and in some embodiments are preferably large-grain emulsions made according to Wilgus German OLS 2,107,118. The monodispersed emulsions are those which comprise silver halide grains having a substantially uniform diameter. Generally, in such emulsions, no more than about 5 percent by number of the silver halide grains smaller than the mean grain size and/or no more than about 5 percent by number of the silver halide grains larger than the mean grain size vary in diameter from the mean grain diameter by more than about 40 percent. Preferred photographic emulsions of this invention comprise silver halide grains, at least 95 percent by weight of the grains having a diameter which is within 40 percent and preferably within about 30 percent of the mean grain diameter. Mean grain diameter, i.e., average grain size, can be determined using conventional methods, e.g., such as projective area, as shown in an article by Trivelli and Smith entitled "Empirical Relations Between Sensitometric and Size-Frequency Characteristics in Photographic Emulsion Series" in *The Photographic Journal*, Volume LXXIX, 1939, pages 330 through 338. The aforementioned uniform size distribution of silver halide grains is a characteristic of the grains in monodispersed photographic silver halide emulsions. Silver halide grains having a narrow size distribution can be obtained by controlling the conditions at which the silver halide grains are prepared using a double run procedure. In such a procedure, the silver halide grains are prepared by simultaneously running an aqueous solution of a silver salt, such as silver nitrate, and an aqueous solution of a water-soluble halide, for example, an alkali metal halide such as potassium bromide, into a rapidly agitated aqueous solution of a silver halide peptizer, preferably gelatin, a gelatin derivative or some other protein peptizer. Suitable methods for preparing photographic silver halide emulsions having the required uniform particle size are disclosed in an article entitled "la: Properties of Photographic Emulsion Grains", by Klein and Moisar, *The Journal of Photographic Science,* Volumne 12, 1964, pages 242 through 251; an article entitled "The Spectral Sensitization of Silver Bromide Emulsions on Different Crystallographic Faces", by Markocki, *The Journal of Photographic Science,* Volume 13, 1965, pages 85 through 89; an article entitled "Studies on Silver Bromide Sols, Part I. The Formation and Aging of Monodispersed Silver Bromide Sols", by Ottewill and Woodbridge, *The Journal of Photographic Science,* Volumn 13, 1965, pages 98 through 103; and an article entitled "Studies on Silver Bromide Sols, Part II. The Effect of Additives on the Sol Particles", by Ottewell and Woodbridge, *The Journal of Photographic Science,* Volume 13, 1965, pages 104 through 107.

Where internal latent image sites have been formed through internal chemical sensitization or the use of metal dopants, the surface of the silver halide grains can be sensitized to a level below that which will produce substantial density in halide grains can be sensitized to a level below that which will produce substantial density in a surface developer (i.e. less than 0.4 when coated, exposed and surface developed as described above). The silver halide grains are preferably predominantly silver bromide grains chemically surface sensitized to a level which would provide a maximum density of at least 0.5 using undoped silver halide grains of the same size and halide composition when coated, exposed and developed as described above.

Surface chemical sensitization can be undertaken using techniques such as those disclosed by Sheppard, Waller et al, McVeigh or Dunn, cited above. The silver halide grains can also be surface sensitized with salts of the nobel metals, such as ruthenium, palladium and platinum. Representative compounds are ammonium chloropalladate, potassium chloropalladate and sodium chloropalladite, which are used for sensitizing in amounts below that which produces any substantial fog inhibition, as described in Smith and Trivelli, U.S. Patent 2,448,060, and as antifoggants in higher amounts, as

described in Trivelli and Smith, U.S. Patent 2,566,245 and U.S. Patent 2,566,263. The silver halide grains can also be chemically sensitized with reducing agents, such as stannous salts (Carroll, U.S. Patent 2,487,850), polyamines, such as diethylene triamine (Lowe et al, U.S. Patent 2,518,698), polyamines, such as spermine (Lowe et al, U.S. Patent 2,521,925), or bis(ß-aminoethyl) sulphide and its water-soluble salts (Lowe et al, U.S. Patent 2,521,926).

The photographic silver halide emulsions of the invention can contain various colloids alone or in combination as vehicles. Suitable hydrophilic materials include such colloidal materials as proteins protein derivatives, cellulose derivatives such as cellulose esters and gelatin, alkali-treated gelatin (cattle bone or hide gelatin) or acid-treated gelatine (pigskin gelatin), gelatin derivatives, acetylated gelatin, phthalated gelatin, polysaccharides such as dextran, gum arabic, zein, casein, pectin, collagen derivatives, collodion, agar-agar, arrowroot and albumin.

The nucleated silver halide emulsions of the invention can be coated as layers on a wide variety of photographic supports to prepare photographic elements. Typical useful photographic supports include polymeric film, wood fibre such as paper, metallic sheet and foil, glass and ceramic supporting elements provided with one or more subbing layers to enhance the adhesive, antistatic, dimensional, abrasive, hardness, frictional, antihalation and/or other properties of the support surface.

Typical useful polymeric film supports are films of cellulose nitrate and cellulose esters, such as cellulose triacetate and diacetate, polystyrene, polyamides, homo- and co-polymers of vinyl chloride, poly(vinyl acetal), polycarbonate, homo- and co-polymers of olefins, such as polyethylene and polypropylene, and polyesters of dibasic aromatic carboxylic acids with divalent alcohols, such as poly(ethylene terephthalate).

Typical useful paper supports are those which are partially acetylated or coated with baryta and/or a polyolefin, particularly a polymer of an $\alpha$-olefin containing 2 to 10 carbon atoms, such as polyethylene, polypropylene, copolymers of ethylene and propylene and the like.

In a preferred form of this invention, the 3,3-disubstituted arylhydrazinophenylthiourea nucleating agents are employed in concentration ranging from 0.1 to 500 mg of adsorbed nucleating agent per mole of silver halide in the emulsion. Preferably, 1 to 100 mg of adsorbed nucleating agent per mole of silver is employed. Optimum concentrations can, of course, vary somewhat from one application to another. Where the 3,3-disubstituted arylhydrazinophenylthiourea nucleating agent is to be adsorbed to the surface of the silver halide grains, it can be adsorbed using the procedures well known to those skilled in the art for adsorbing sensitizing dyes, such as cyanine and merocyanine dyes, to the surface of silver halide grains.

A simple exposure and development process can be used to form a direct-positive image. In one embodiment, a photographic element comprising at least one layer of a silver halide emulsion as described above can be imagewise exposed and then developed in a silver halide surface developer.

It is understood that the term "surface developer" encompasses those developers which will reveal the surface latent image on a silver halide grain, but will not reveal substantial internal latent image in an internal image-forming emulsion, and under the conditions generally used develop a surface-sensitive silver halide emulsion. The surface developers can generally utilize any of the conventional photographic silver halide developing agents or reducing agents, but the developing bath or composition is generally substantially free of a silver halide solvent (e.g. water-soluble thiocyanates, water-soluble thioethers, thiosulphates and ammonia) which will disrupt or dissolve the grain to reveal substantial internal image. Low amounts of excess halide are sometimes desirable in the developer or incorporated in the emulsion as halide-releasing compounds, but high amounts of iodide or iodide-releasing compounds are generally avoided to prevent substantial disruption of the grain. Typical silver halide developing agents which can be used in the developing compositions of this invention include hydroquinones, catechols, aminophenols, 3-pyrazolines, ascorbic acid and its derivatives, reductones, phenylenediamines and the like, or combinations thereof. Illustrative of useful surface developers are those disclosed in Ives U.S. Patent 2,563,785; Evans U.S. Patent 3,761,276; Knott et al U.S. Patent 2,456,953 and Juoy U.S. Patent 3,511,662.

Where the developing agents are initially partly or entirely incorporated in the photographic elements, the remaining components (e.g., water, activators to adjust pH and preservatives) conventionally present in surface developers constitute what is commonly referred to as an activator solution. Except for the omission of part or all of the developing agent, activator solutions are idential to developer solutions in composition and are employed when the developing agent is incorporated in the photographic element. Subsequent references herein to developing compositions are inclusive of both developer and activator solutions.

Photographic elements containing monosubstituted acylhydrazinophenylthioureas are preferably processed with developing compositions having pH at our above 13.5. At lower pH levels the nucleating activity of monosubstituted acylhydrazinophenylthioureas is significantly diminished.

We have now found that 3,3-disubstituted acylhydrazinophenylthioureas retain to a relatively high degree their nucleating activity when employed with developing compositions at pH levels down to and below 12.0. In addition to being useful at higher pH levels conventionally employed with monosubstituted acylhydrazinophenylthioureas, typically in the range of from 13.5 to 13.9, the 3,3-disubstituted acylhydrazinophenylthioureas are useful at pH levels as low as 11.8 and, depending upon

the specific form of the photographic element employed, even lower. It is frequently desirable to lower developing composition pH levels to reduce the potential hazard which the higher pH levels entail if the developing compositions are carelessly or otherwise improperly handled. For such applications, it is specifically preferred to employ developing compositions in the lower pH ranges, of from about 12.0 to 13.0, in processing photographic elements according to this invention containing 3,3-disubstituted acylphenylhydrazinophenylthioureas.

The developing compositions used in the process of this invention can contain certain antifoggants and development restrainers, or, optionally, they can be incorporated in layers of the photographic element. For example, in some applications, improved results can be obtained when the direct-positive emulsions are processed in the presence of certain antifoggants, as disclosed in Stauffer U.S. Patent 2,497,917.

Typical useful antifoggants include benzotriazoles, such as benzotriazole, 5-methylbenzotriazole, and 5-ethylbenzotriazole, benzimidazoles such as 5-nitrobenzimidazole, benzothiazoles such as 5-nitrobenzothiazole and 5-methylbenzothiazole, heterocyclic thiones such as 1-methyl-2-tetrazoline-5-thione, triazines such as 2,4-dimethylamino-6-chloro-5-triazine, benzoxazoles such as ehtylbenzoxazole and pyrroles such as 2,5-dimethylpyrrole.

In certain embodiments, good results are obtained when the elements are processed in the presence of high levels of the antifoggants mentioned above. When antifoggants such as benzotriazoles are used, good results can be obtained when the processing solution contains 500 mg to 100 grams per litre, and preferably 1 to 5 grams per litre. When the antifoggants are incorporated in the photographic element, concentrations of 5 to 500 mg. per mole of silver halide and preferably concentrations of 10 to 150 mg per mole of silver halide, are employed. Optimum antifoggant concentrations vary with the particular photographic element and processing solution.

A variety of features that can be used in conjunction with the invention are disclosed in *Research Disclosure*, Volume 176, December 1978, Item 17643, the disclosure of which is herein incorporated by reference, particularly Paragraph II, Emulsion Washing; Paragraph IV, Spectral Sensitization and Desensitization; Paragraph V, Brighteners; Paragraph VI, Antifoggants and Stabilizers; Paragraph VIII, Adsorbing and Scattering Materials; Paragraph X, Hardeners; Paragraph XI, Coating Aids; Paragraph XII, Plasticizers and Lubricants; Paragraph XIII, Antistatic Layers; Paragraph XIV, Methods of Addition, Paragraph XV, Coating and Drying Procedures; Paragraph XVI, Matting Agents; Paragraph XVIII, exposure; Paragraph XX, Developing Agents; and Paragraph XXI, Development Modifiers.

The 3,3-disubstituted acylhydrazinophenylthiourea nucleating agents can be used with other conventional nucleating agents. In a preferred form one or a combination of 3,3-disubstituted acylhydrazinophenylthiourea nucleating agents are employed at a concentration of up to about 200 mg per mole of silver in combination with a conventional substituted hydrazine type nucleating agent which is present in a concentration of from about 200 mg to about 2 grams per mole of silver. Conventional nucleating agents that can be used in combination with the 3,3-disubstituted acylhydrazinophenylthiourea nucleating agents employed in the invention are disclosed in U.S. Patents 2,011,391; 2,012,443; 3,719,494; 3,734,738; 3,615,615; 3,759,901; 4,115,192; 4,030,925; 4,080,207; 3,708,302 and 3,869,286.

The silver halide emulsions of the invention can be spectrally sensitized with cyanine, merocyanine, and other polymethine dyes and supersensitizing combinations thereof well known in the art. Spectral sensitizers in conventional surface-sensitive emulsions are comparably effective in the emulsions of this invention. In general, they enhance nucleation. Non-ionic, zwitterionic and anionic spectral sensitizers are preferred. Particularly effective are carboxy-substituted merocyanine dyes of the thiohydantoin type described by Stauffer and Spence U.S. Patent 2,490,758.

Supersensitizing dye combinations having polarographic oxidation potentials ($E_{ox}$) of about 0.3 to 0.9 volt can be used in the emulsion of the invention. Such combinations are described in U.S. Patents 2,075,048; 2,313,922; 2,533,426; 2,704,714; 2,704,717; 2,688,545 and 3,672,898. Particularly effective supersensitizing combinations are described in U.S. Patents 3,397,060; 2,688,545; 2,701,198 and 2,973,264.

Photographic elements containing the nucleated silver halide emulsions of the invention are preferably colour photographic elements which form dye images through the selective destruction, formation or physical removal of dyes. Such photographic elements can produce dye images through the selective destruction of dyes or dye precursors, such as conventional silver-dye-bleach processes. The photographic elements can produce dye images through the selective formation of dyes, such as by reacting (coupling) a colour-developing agent (e.g., a primary aromatic amine) in its oxidized form with a dye-forming coupler. The dye-forming couplers can be incorporated in the photographic elements. In one form, the dye-forming couplers can be chosen to form subtractive primary (i.e., yellow, magenta and cyan) image dyes and are nondiffusible, colourless couplers, such as two- and four-equivalent couplers of the open chain ketomethylene, pyrazolone, pyrazolotriazole, pyrazolobenzimidazole, phenol and naphthol type hydrophobically ballasted for incorporation in high-boiling organic (coupler) solvents. The photographic elements can incorporate known alkali-soluble ballasted couplers. The photographic elements can be adapted to form non-diffusible image dyes using dye-forming couplers in developers. The dye-forming couplers upon coupling can release photographically useful fragments, such

as development inhibitors or accelerators, bleach accelerators, developing agents, silver halide solvents, toners, hardeners, fogging agents, anti-foggants, competing couplers, chemical or spectral sensitizers and densensitizers. The photographic elements can also contain coloured dye-forming couplers, such as those employed to form integral masks for negative colour images. Also the photographic elements can produce dye images through the selective removal of dyes. Negative or positive dye images can be produced by the immobilization or mobilization of incorporated colour-providing substances as a function of exposure and development.

The nucleated silver halide emulsions of the invention are particularly useful in photographic elements used in image transfer systems.

Suitable image transfer systems include colloid transfer systems, as illustrated by Yutzy et al, U.S. Patents 2,596,756 and 2,716,059; imbibition transfer systems, as illustrated by Minsk, U.S. Patent 2,882,156; and colour image transfer systems, as illustrated by *Research Disclosure*, Volume 151, November 1976, Item 15162, and Volume 123, July 1974, Item 12331.

Image transfer film units in accordance with this invention comprise:

(1) a photographic element comprising a support having thereon at least one silver halide emulsion layer containing radiation-sensitive internal latent image silver halide grains and a 3,3-disubstituted arylhydrazinophenylthiourea nucleating agent as described above, the emulsion layer having within or in contact thereto an image dye-providing material,

(2) an image-receiving means, which can be located on a separate support and superposed or adapted to be superposed on the photographic element or, preferably, can be coated as a layer in the photographic element, positioned to receive image dye,

(3) an alkaline processing composition,

(4) means containing and adapted to release the alkaline processing composition into contact with the emulsion layer, and

(5) a silver halide developing agent located in at least one of the photographic element and alkaline processing composition so that the processing composition and developing agent, when brought together, form a silver halide surface developer.

In highly preferred embodiments, the film units of this invention contain a support having thereon a layer containing a blue-sensitive emulsion and in contact therewith a yellow image dye-providing material, a red-sensitive silver halide emulsion and in contact therewith a cyan image dye-providing material, and a green-sensitive emulsion and in contact therewith a magenta image dye-providing material, and preferably all of said image dye-providing materials are initially immobile image dye-providing materials.

The terms "diffusible" (or "mobile") and "immobile" or "nondifusible"), as used herein, refer to compounds which are incorporated in the photographic element and, upon contact with an alkaline processing solution, are substantially diffusible or substantially immobile, respectively, in the hydrophilic colloid layers of a photographic element.

The term "image dye-providing material", as used herein, refers to those compounds which are employed to form dye images in photographic elements. These compounds include dye developers, shifted dyes, color couplers, oxichromic compounds and dye redox releasers. Preferred image dye-providing materials are disclosed in U.S. Patents 3,698,897; 3,725,062; 3,928,312; 4,053,312 and 4,076,529; Canadian Patent 602,607; German OLS 2,505,248; German OLS 2,613,005 and German OLS 2,729,820.

In one preferred embodiment of image transfer unit the receiver layer is coated on the same support with the photosensitive silver halide emulsion layers, the support is a transparent support, an opaque layer is positioned between the image-receiving layer and the photosensitive silver halide layer, and the alkaline processing composition contains an opacifying substance, such as carbon or a pH-indicator dye which is discharged onto the film unit between a dimensionally stable support or cover sheet and the photosensitive element.

Preferred colour image transfer film units and system features, are more specifically disclosed in *Research Disclosure*, Volume 151, November 1976, Item 15162.

Preparation 1

Preparation of 1-[4-(2-formyl-hydrazino)-phenyl]-3,3-dimethylthiourea (NA-1). 1-Formyl-2-(4-aminophenyl)hydrazine (1.51 g, 0.01 mole) and triethylamine (1.0 g. 0.01 mole) were mixed with dry acetonitrile (30 ml). The mixture was kept under a nitrogen atmosphere and a solution of dimethylthiocarbamoyl chloride (1.24 g, 0.01 mole) in acetonitrile (10 ml) was added dropwise at 25°C. After the addition was complete, the reaction mixture was chilled in ice, then filtered. The solid was washed with ethanol and allowed to dry. The material was stirred in water (40 ml) and heated to 60°C to remove any hydrochloride salts. The aqueous mixture was filtered; the solid was washed with diethyl ether and dried. This gave 0.60 g (25 percent) of product as a pale tan powder, m.p. 187—189°C.

Preparation 2

Preparation of 1-[4-(2-acetylhydrazino)-phenyl]-3,3-dimethylthiourea (NA-5). The procedure for the preparation of NA-1 in Preparation 1 was followed with 1-acetyl-2-(4-aminophenyl)-hydrazine

(0.82 g. 0.005 mole), dimethylthiocarbamoyl chloride (0.62 g, 0.005 mole) and N,N-diisopropylethylamine (0.65 g, 0.005 mole). Yield 0.30 g (24 percent), m.p. 187—189°C.

Preparation 3

Preparation of 1-[4-(2-formylhydrazino)-phenyl]-3,3-dibenzyl-thiourea(NA-11), 4-(2-Formylhydrazino)phenyl isothiocyanate was first prepared in the following manner: 1-formyl-2-(4-aminophenyl)-hydrazine (1.1 g, 0.0075 mole) was dissolved in dry acetone (75 ml) and the resulting solution was cooled to −78°C in a dry ice-acetone bath. The reaction mixture was stirred and kept under a nitrogen atmosphere. A solution of 1,1′-thiocarbonyldiimidazole (1.35 g, 0.0075 mole) in dry acetone (75 ml) was added dropwise to the reaction mixture. After the addition was complete, the mixture was stirred for 30 minutes at −78°C, then allowed to warm to 25°C. The solvent was removed at reduced pressure. The remaining solid residue was stirred in water at room temperature. The solid was filtered off, washed with water, then ether, and allowed to dry. The crude product was recrystallized from acetone to give 0.80 g (55 percent) of a white crystalline powder, m.p. 178—180°C.

Dibenzylamine (0.20 g, 0.001 mole) and 4-(2-formylhydrazino)phenyl isothiocyanate (0.19 g, 0.001 mole) were mixed in ethanol (25 ml) and the resulting mixture was heated to reflux for 20 minutes. The reaction mixture was cooled to room temperature, then was chilled in ice. After scratching the flask walls, a white crystalline solid was separated out of solution. The solid was filtered off, washed thoroughly with diethyl ether, and allowed to dry. This gave 0.31 g (80 percent) of product as a white crystalline powder, m.p. 174—176°C.

Preparation 4

Preparation of 1-[4-(2-acetylhydrazino)-phenyl]-3,3-dibenzylthiourea (NA-12). 4-(2-Acetylhydrazino)phenyl isothiocyanate was prepared as described for NA-11 in Preparation 3 with 1-acetyl-2-(4-aminophenyl)hydrazine (1.23 g, 0.0075 mole) and 1,1′-thiocarbonyldiimidazole (1.35 g, 0.0075 mole). Yield 1.20 g (77 percent), m.p. 172—174°C.

Dibenzylamine (0.20 g, 0.001 mole) and 4-(2-acetylhydrazino)phenyl isothiocyanate (0.21 g, 0.001 mole) were reacted according to the procedure described for NA-11 in Preparation 3. Yield 0.35 g (88 percent), m.p. 207—209°C.

Preparation 5

Preparation of 1-[4-(2-formylhydrazino)-phenyl]-3-methyl-3-phenylthiourea (NA-14). N-Methylaniline (0.11 g, 0.001 mole) and 4-(2-formylhydrazino)phenyl isothiocyanate (0.19 g, 0.001 mole) were reacted according to the procedure described for NA-11 in Preparation 3. Yield 0.15 g (50 percent), m.p. 137—139°C.

Preparation 6

Preparation of 1-[4-(2-formylhydrazino)-phenyl]-3,3-dibutylthiourea (NA-15). Di-n-butylamine (0.13 g, 0.001 mole) and 4-2-formylhydrazino)phenyl isothiocyanate (0.19 g, 0.001 mole) were reacted according to the procedure described for NA-11 in Preparation 3. Yield 0.10 g (31 percent), m.p. 139—141°C.

Preparation 7

Preparation of 4-[4-(2-formylhydrazino)-phenylthiocarbamoyl] morpholine (NA-21). Morpholine (0.087 g, 0.001 mole) and 4-(2-formylhydrazino)phenyl isothiocyanate (0.19 g. 0.001 mole) were reacted according to the procedure described for NA-11 in Preparation 3. Yield 0.22 g (79 percent), m.p.204—206°C.

Preparation 8

Preparation of 1-[4-(2-formylhydrazino)-phenylthiocarbamoyl] piperidine (NA-32). Piperidine (0.085 g, 0.001 mole) and 1-(2-formylhydrazino)phenyl isothiocyanate (0.19 g, 0.001 mole) were reacted according to the procedure described for NA-11 in Preparation 3. Yield 0.14 g (50 percent), m.p. 174—176°C.

Preparation 9

Preparation of 1-[4-(2-formylhydrazino)-phenylthiocarbamoyl] pyrrolidine (NA-26). Pyrrolidine (0.071 g, 0.001 mole) and 1-(2-formylhydrazino)phenyl isothiocyanate (0.19 g, 0.001 mole) were reacted according to the procedure described for NA-11 in Preparation 3. Yield 0.15 g (58 percent), m.p. 199—201°C.

Preparation 10

Preparation of 1-[4-2-formylhydrazino)-phenylthiocarbamoyl]-3-pyrroline (NA-25). 3-Pyrroline (0.069 g, 0.001 mole) and 1-(2-formylhydrazino)phenyl isothiocyanate (0.19 g, 0.001 mole) were reacted according to the procedure described for NA-11 in Preparation 3. Yield 0.18 g (69 percent), m.p. 218—220°C.

Example 1

Photographic Comparisons

A photographic multicolour image transfer element was prepared by coating onto a poly(ethylene terephthalate) film support to produce the indicated layer arrangement. (Coverages are expressed in $g/m^2$ unless otherwise specified.)

| |
|---|
| Layer 9 — Overcoat layer of gelatin (0.86) and a latex mordant, poly(styrene-*co*-N-vinylbenzyl-N-benzyl-N,N'-dimethylammonium sulphate-*co*-divinylbenzene (0.11) |
| Layer 8 — Blue-sensitive internal image gelatin (0.81) silver bromide (0.75) emulsion; sodium 5-octadecylhydroquinone-2-sulphonate (12 g/mole Ag); and the same prior art nucleating agent present in Layer 2 (10 mg/mole AG) |
| Layer 7 — Gelatin (1.08) and a yellow redox dye-releaser of the type described in U.S. Patent 4,013,633 (0.65) |
| Layer — Interlayer of gelatin (0.97) and didodecylhydroquinone (0.70) |
| Layer 5 — Green-sensitive internal image gelatin (0.81) silver bromide (0.75) emulsion; sodium 5-octadecylhydroquinone-2-sulphonate (12 g/mole Ag); and the same prior art nucleating agent present in Layer 2 (10 mg/mole Ag) |
| Layer 4 — Gelatin (1.08) and a magenta redox dye-releaser of the type described in U.S. Patent 3,954,476 (0.54) |
| Layer 3 — Interlayer of gelatin (0.97) and didodecylhydroquinone (0.70) |
| Layer 2 — Red-sensitive internal image gelatin (1.08) silver bromide (0.75) emulsion; sodium 5-octadecylhydroquinone-2-sulphonate (12 g/mole Ag); and prior art nucleating agent 1-[4-(2-formylhydrazino)phenyl]-3-methylthiourea (C-1) (8 mg/mole Ag) |
| Layer 1 — Gelatin (1.08) and a cyan redox dye-releaser of the type disclosed in U.S. Patent 3,942,987 (0.54) |
| ////////////////////////////////// SUPPORT ////////////////////////////////////// |

A second element was identically prepared, except that the three emulsion layers each contained the nucleating agent 1-[4-(2-formylhydrazino)phenyl]-3,3-dimethylthiourea (NA-1) substituted at the same concentration in lieu of the prior art nucleating agent 1-[4-(2-formylhydrazino)phenyl]-3-methylthiourea (C-1).

Each element was exposed to a multicolour graduated density test object for 0.5 second and soaked for 15 seconds in an activator at 29°C, identified as Activator Solution A (described below in Table II).

TABLE II

| | |
|---|---|
| Benzyl alcohol | 10 ml |
| 5-methylbenzotriazole | 1 g |
| 11-aminoundecanoic acid | 2 g |
| 6-aminohexanoic acid | 10 g |
| 0.5 N potassium hydroxide to pH 13.5 | 1 litre |

The element was then laminated to a dry image receiver sheet for two minutes, then peeled apart, and the receiver was washed with water.

The receiver sheet comprised the following layers coated on a polyethylene-coated paper support.

| |
|---|
| Layer 2 — An overcoat layer of polyvinyl alcohol (0.11) |
| Layer 1 — A mordant layer of gelatin (2.28) and a latex poly(styrene-co-N-vinylbenzyl-N-benzyl-N,N-dimethylammonium sulphate-*co*-divinylbenzene) (2.28), 4-hydroxymethyl-4-methyl-1-phenyl-3-pyrazolidone (0.16) |
| ////////////////////////////////// SUPPORT ////////////////////////////////////// |

The $D_{max}$ and $D_{min}$ of the sensitometric curves are shown in Table III.

TABLE III

| Nucleating Agent | $D_{max}$ Blue | $D_{max}$ Green | $D_{max}$ Red | $D_{min}$ Blue | $D_{min}$ Green | $D_{min}$ Red |
|---|---|---|---|---|---|---|
| 1-[4-(2-formylhydrazino)-phenyl]-3-methylthiourea (C-1) | 1.88 | 2.15 | 0.35 | 0.25 | 0.34 | 0.19 |
| 1-[4-(2-formylhydrazino)-phenyl]-3,3-dimethylthiourea (NA-1) | 2.26 | 2.45 | 2.40 | 0.38 | 0.54 | 0.35 |

The data show that nucleating agent NA-1 is a more active nucleating agent than the 3-mono-substituted nucleating agent C-1 as indicated by the higher $D_{max}$ and $D_{min}$ values at the same concentration.

Example 2

A series of photographic single color image transfer elements were prepared having the following layers coated on a black opaque poly(ethylene terephthalate) film support. The coatings differed only in the type of nucleating agent in the emulsion layer. The prior art nucleating agent 1-[4-(2-formylhydrazino)phenyl]-3-phenylthiourea (C-2) of U.S. Patent 4,030,925 was coated at $4.5 \times 10^{-5}$ mole/mole Ag. The nucleating compounds used in the invention were substituted at the same concentration in otherwise identical elements. The elements exhibited the following layer arrangement.

Layer 3 — An overcoat layer of gelatin (0.86) and a latex mordant poly(styrene-*co*-N-vinylbenzyl-N-benzyl-N,N-dimethylammonium sulphate-*co*-divinyl-benzene) (0.11)

Layer 2 — A green-sensitive internal-image silver bromide (0.43 Ag) gelatin (1.1) emulsion including 12 g/mole sodium 5-octadecylhydroquinone-2-sulphonate and the nucleating agent

Layer 1 — Gelatin (1.34) and magenta redox dye-releaser (0.48) of the type disclosed in Fernandez U.S. Patent 4,135,929

////////////////////////////////// SUPPORT //////////////////////////////////////

The elements were exposed as described in Example 1, given a 10 second soak in an activator, either Activator Solution A (described above in Table II) or Activator Solution B (described below in Table IV) at 29°C, and then laminated to a dry image receiver sheet for two minutes, peeled apart, and the receiver sheet washed with water.

TABLE IV

| | |
|---|---|
| Benzyl alcohol | 8 ml |
| 5-methylbenzotriazole | 1 g |
| 11-aminoundecanoic acid | 2 g |
| KBr | 4 g |
| 0.5 N potassium hydroxide to pH 13.5 | 1 litre |

The receiver sheet was comprised of a mordant layer coated on a polyethylene-coated paper support, as indicated below.

A mordant layer of gelatin (1.71) and poly(N-vinylimidazole-*co*-3-$\beta$-hydroxyethyl-1-vinylimidazolium chloride) (2.28), 4-hydroxymethyl-4-methyl-1-phenyl-3-pyrazolidinone (0.16)

////////////////////////////////// SUPPORT //////////////////////////////////////

The activity levels of the nucleating agents are compared in Table V, which compares relative nucleating activity of compounds of this invention with prior art nucleating agent C-2. The activity rating value is based upon the concentration of nucleating agent that is required to match as closely as possible the characteristic curve (that is, similar $D_{max}$, contrast, speed and $D_{min}$) as prior art nucleating agent C-2. For example, with C-2 assigned an activity rating of 1.0, a nucleating agent with a rating of 2.0 is twice as active i.e., only one-half the concentration of nucleating agent on a molar weight basis is required to give the same relative curve shape as compound C-2. Acylhydrazinophenylthioureas having a formyl acyl group are more active than otherwise comparable nucleating agents.

TABLE V

Relative Nucleation Activity

| Nucleating Agent | | Activity Rating | |
|---|---|---|---|
| | | Solution A | Solution B |
| C—2 | 1-[4-(2-formylhydrazino)phenyl]-3-phenylthiourea | 1.0 | 1.0 |
| NA—1 | 1-[4-(2-formylhydrazino)phenyl]-3,3-dimethylthiourea | 3.33 | 2.12 |
| NA—11 | 1-[4-(2-formylhydrazino)phenyl]-3,3-dibenzylthiourea | 2.5 | 1.5 |
| NA—14 | 1-[4-(2-formylhydrazino)phenyl]-3-methyl-3-phenylthiourea | 6.33 | 4.25 |
| NA—21 | 4-[4-(2-formylhydrazino)phenylthiocarbamoyl]morpholine | 4.33 | 2.63 |
| NA—32 | 1-[4-(2-formylhydrazino)phenylthiocarbamoyl]piperidine | 5.33 | 3.63 |
| NA—26 | 1-[4-(2-formylhydrazino)phenylthiocarbamoyl]pyrrolidine | 5.67 | 3.5 |
| NA—25 | 1-[4-(2-formylhydrazino)phenylthiocarbamoyl]-3-pyrroline | 3.17 | 1.88 |

Example 3

A comparison between nucleating agent C—1 at a concentration of $1.8 \times 10^{-4}$ mole/mole Ag and a nucleating agent used in the invention was made utilizing an activator solution having a pH of only 12.0.

Single colour image transfer elements were prepared having the following layer arrangement coated on a black opaque poly(ethylene terephthalate) film support.

| |
|---|
| Layer 3 — An overcoat layer of gelatin (0.86) and a latex mordant poly(styrene-*co*-N-vinylbenzyl-N-benzyl-N,N-dimethylammonium sulphate-*co*-divinyl-benzene (0.11) |
| Layer 2 — A green-sensitive internal image silver bromide (1.07 Ag) gelatin (1.61) emulsion including 12 g/mole sodium 5-octadecylhydroquinone-2-sulphonate and the nucleating agent |
| Layer 1 — Gelatin (1.61) and a magenta redox dye-releaser E (0.54) of the type disclosed by U.S. Patent 3,954,475 |
| ////////////////////////////////// SUPPORT ////////////////////////////////////// |

The elements were exposed for 0.2 second through a graduated density test object, soaked for 40 seconds in Activator Solution C (described below in Table VI) at 22°C, and then laminated to a dry image receiver sheet for 3 minutes, peeled apart and the receiver sheet washed with water.

TABLE VI

Activator Solution C

| | |
|---|---|
| $K_3PO_4$ | 60 g |
| benzyl alcohol | 10 ml |
| 5-methylbenzotriazole | 1 g |
| 11-aminoundecanoic acid | 2 g |
| distilled water to pH 12.0 | 1 litre |

The receiver sheet was comprised of a mordant layer coated over a gelatin layer (0.86) on a polyethylene-coated paper support. The mordant layer consisted of gelatin (2.28) and poly(styrene-*co*-N-vinylbenzyl-N-benzyl-N,-dimethylammonium sulphate-*co*-divinylbenzene) (2.28) and 4-hydroxymethyl-4-methyl-1-phenyl-3-pyrazolidione (0.22).

The results are set forth below in Table VII.

TABLE VII

Relative Nucleation Activity

| Nucleator | Activity Rating Solution C |
|---|---|
| 1-[4-(2-formylhydrazino)phenyl]-3-methylthiourea (C—1) | 1.0 |
| 1-[4-(2-formylhydrazino)phenyl]-3,3-dimethylthiourea (NA—1) | 2.1 |

This demonstrates the superior nucleation activity of the nucleating agents used in the invention as compared with prior art acylhydrazinophenylthiourea nucleating agents at lower pH levels.

Example 4

To provide a direct comparison of a mono-substituted acetyl hydrazide nucleating agent according to Leone et al U.S. Patent 4,030,925 and an otherwise identical di-substituted acetyl hydrazide nucleating agent used in the invention, single colour image transfer elements were prepared having the following layer arrangement coated on a black opaque poly(ethylene terephthalate) support.

| |
|---|
| Layer 3 — An overcoat layer of gelatin (1.29) containing didodecyl hydroquinone (0.23) and 1-phenyl-2-pyrazolin-3-yl-N-methyl-[2-(N-methyltrifluoroacetamidomethyl)-4-*p*-toluene-sulphonamidophenyl]carbamate (0.32) |
| Layer 2 — A green-sensitive internal image silver bromide (0.48) gelatin (1.29) emulsion including 6 g/Ag mole sodium 5-octadecylhydroquinone-2-sulfonate and $1.12 \times 10^{-4}$ mole/Ag mole of the nucleating agent |
| Layer 1 — Gelatin (1.29), a magenta redox dye-releaser (0.48) of the type disclosed in Fernandez U.S. Patent 4,135,929 and sodium 5-octadecylhydroquinone-2-sulphonate (0.05) |
| ////////////////////////////////SUPPORT//////////////////////////////////// |

The elements were exposed for 0.5 second to a multicolour graduated density test object, soaked for 13 seconds at 29°C in an activator solution similar to Activator Solution B (but with no benzyl alcohol), then laminated to a dry image receiver sheet for two minutes, peeled apart and the receiver sheet washed with water. The receiver sheet was of the type described in *Research Disclosure*, Vol. 185, September 1979, Item 18534.

The nucleating agents employed and a comparison of the maximum and minimum densities produced are set forth below in Table VIII.

TABLE VIII

| Nucleating Agent | Dye Densities $D_{max}$ | $D_{min}$ |
|---|---|---|
| 1-[4-(2-acetylhydrazino)phenyl]-3-benzylthiourea (C—3) | 0.92 | 0.10 |
| 1-[4-(2-acetylhydrazino)phenyl]-3,3-dibenzylthiourea (NA—12) | 1.26 | 0.10 |

**Claims**

1. A silver halide emulsion comprising silver halide grains capable of forming an internal latent image, the silver halide grains having adsorbed on their surfaces an acylhydrazinophenylthiourea nucleating agent, characterised in that the nucleating agent has the formula:

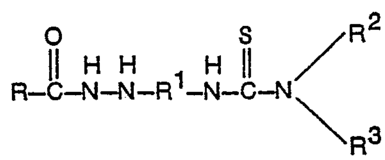

wherein

R is hydrogen, cycloalkyl which may be substituted with halogen, cyano or alkoxy groups or alkyl which may be substituted with halogen, alkoxy or phenyl groups;

$R^1$ is a phenylene group or a phenylene group substituted with alkyl, halogen or alkoxy; and

$R^2$ and $R^3$ are independently

a) an alkyl, haloalkyl, alkoxyalkyl or phenylalkyl group having from 1 to 18 carbon atoms;

b) a cycloalkyl group which may be substituted with halogen, cyano or alkoxy groups;

c) a phenyl group having a positive Hammett sigma value-derived electron-withdrawing characteristic less than +0.50; or

d) a naphthyl group; or

$R^2$ and $R^3$ together complete a heterocyclic group having a 5- or 6-membered ring which may have at least one additional hetero atom selected from nitrogen, oxygen, sulfur and selenium; the alkyl groups, except as otherwise indicated, having 1 to 6 carbon atoms; and the cycloalkyl groups having 3 to 10 carbon atoms.

2. A silver halide emulsion according to claim 1 which contains from 0.1 to 500 mg of said nucleating agent per mole of silver halide.

3. A silver halide emulsion according to claim 2 which contains from 1 to 100 mg of said nucleating agent per mole of silver halide.

4. A silver halide emulsion according to claim 1, 2 or 3 wherein the nucleating agent has the formula:

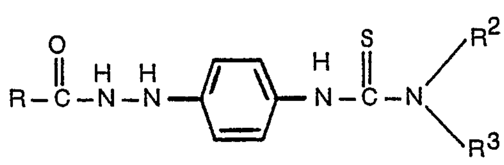

wherein

R is H or methyl and

$R^2$ and $R^3$ are independently an alkyl group having 1 to 6 carbon atoms, a phenylalkyl group wherein the alkyl has 1 to 6 carbon atoms or a phenyl group having a positive Hammett sigma value-derived electron-withdrawing characteristic less than +0.50; or

$R^2$ and $R^3$ together complete a saturated heterocyclic nucleus having a 5- or 6-membered ring containing as additional hetero atom at least one of nitrogen, oxygen, sulfur and selenium.

5. A silver halide emulsion according to claim 4, each of $R^2$ and $R^3$ being methyl, benzyl or phenyl.

6. A silver halide emulsion according to claim 4, $R^2$ and $R^3$ together completing said heterocyclic ring and said ring containing an additional hetero ring atom selected from N, O, S and Se.

7. A silver halide emulsion according to claim 4, $R^2$ and $R^3$ together completing a morpholino, a pyrrolidyl, pyrrolyl or piperidino ring.

8. A silver halide emulsion according to claim 4, said nucleating agent being

1-[4-(2-formylhydrazino)phenyl]-3,3-dimethylthiourea,
1-[4-(2-formylhydrazino)phenyl]-3,3-dibenzylthiourea,
1-[4-(2-acetylhydrazino)phenyl]-3,3-dibenzylthiourea,
1-[4-(2-formylhydrazino)phenyl]-3-methyl-3-phenylthiourea,
4-[4-(2-formylhydrazino)phenylthiocarbamoyl]morpholine,
1-[4-(2-formylhydrazino)phenylthiocarbamoyl]-3-pyrroline,
1-[4-(2-formylhydrazino)phenylthiocarbamoyl]pyrrolidine or
1-[4-(2-formylhydrazino)phenylthiocarbamoyl]piperidine.

9. A silver halide emulsion according to any of claims 1 to 8 wherein the emulsion contains an antifoggant.

10. A silver halide emulsion according to claim 9 wherein the antifoggant is a benzothiazole at a concentration of 5 to 500 mg per mole of silver halide.

11. A photographic element comprising a support and a layer thereon of a silver halide emulsion characterised in that it comprises a silver halide emulsion according to any of claims 1 to 10.

12. A photographic element according to claim 11 wherein said layer has associated therewith a dye image-providing compound.

13. A photographic element according to claim 12 wherein said compound is a redox dye-releaser, said dye being of a colour complementary to the colour to which the silver halide is sensitive.

14. A photographic element according to claim 13 comprising three said layers sensitive respectively to blue, green and red.

15. An image-transfer film unit which comprises a photographic element according to any of claims 11 to 14, a dye image-receiving layer on said support or on another support, a reservoir of alkaline processing fluid and a surface silver halide developing agent.

16. A direct positive process for producing a visible image in an imagewise exposed photographic element characterised by processing an element according to any of claims 11 to 15 in an aqueous alkaline processing composition in the presence of a surface developing agent.

17. A direct positive process according to claim 16 wherein the composition is at a pH in the range of from 13.9 to 11.8.

18. A direct positive process according to claim 16 or 17 wherein the aqueous alkaline processing composition contains an antifoggant.

19. A direct positive process according to claim 18 wherein the aqueous alkaline processing composition contains a benzotriazole antifoggant at a concentration of 500 mg to 10 grams per litre.

**Revendications**

1. Emulsion aux halogénures d'argent qui comprend des grains d'halogénures d'argent apte à former une image latente interne, ces grains d'halogénures d'argent contenant, adsorbé à leur surface, un agent de nucléation de la classe des acylhydrazinophénylthiourées, cette émulsion étant caractérisée en ce que l'agent de nucléation correspond à la formule suivante:

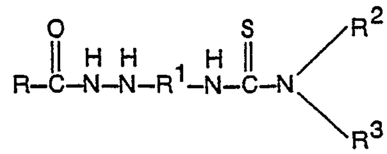

où:

R représente un atome d'hydrogène ou un radical cycloalkyle, halogénocycloalkyle, alcoxycycloalkyle ou cyanocycloalkyle ou un radical alkyle que peut être substitue par des radicaux halogéno, alcoxy ou phényle,

$R^1$ représente un groupe phénylène ou un groupe phénylène substitué par un radical alkyle, halogéno, ou alcoxy, et

$R^2$ et $R^3$ représentent chacun,

a) un radical alkyle, halogénoalkyle, alcoxyalkyle ou phénylalkyle de 1 à 18 atomes de carbone,

b) un groupe cycloalkyle qui peut être substitué par des groupes halogéno, cyano ou alcoxy,

c) un groupe phényle possédant une caractéristique d'accepteur d'électrons dérivée de la constante de Hammett positive inférieure à +0,05, ou,

d) un groupe naphtyle, ou,

$R^2$ et $R^3$, considérés ensemble, complètent un groupement hétérocyclique à 5 ou 6 chaînons, qui peut avoir au moins un hétéroatome supplémentaire choisi parmi l'azote, l'oxygène, le soufre et le sélénium,

les groupes alkyle, sauf indication contraire, comprenant 1 à 6 atomes de carbone et les groupes cycloalkyle comprenant 3 à 10 atomes de carbone.

2. Emulsion aux halogénures d'argent conforme à la revendication 1, caractérisée en ce qu'elle contient 0,1 mg à 500 mg d'agent de nucléation par mole d'halogénures d'argent.

3. Emulsion aux halogénures d'argent conforme à la revendication 2, caractérisée en ce qu'elle contient 1 mg à 100 mg d'agent de nucléation par mole d'halogénures d'argent.

4. Emulsion aux halogénures d'argent conforme à l'une des revendications 1, 2 ou 3, caractérisée en ce que l'argent de nucléation correspond à la formule suivante:

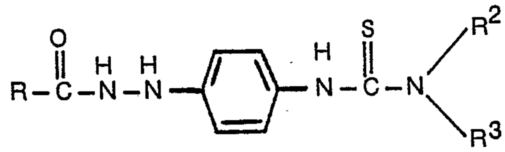

où:

R représente un atome d'hydrogène ou le radical méthyle, et

$R^2$ et $R^3$ représentent chacun un groupe alkyle de 1 à 6 atomes de carbone, un groupe phénylalkyle dont le radical alkyle contient 1 à 6 atomes de carbone ou un groupe phényle dont la caractéristique d'accepteur d'électrons dérivée de la constante de Hammett est positive et inférieure à +0,50, ou,

$R^2$ et $R^3$, considérés ensemble, complètent un groupement hétérocyclique saturé à 5 ou 6 chaînons qui contient comme hétéroatome supplémentaire au moins un atome d'azote, d'oxygène, de soufre ou de sélénium.

5. Emulsion aux halogénures d'argent conforme à la revendication 4, caractérisée en ce que chaque groupement $R^2$ et $R^3$ représente le groupe méthyle, benzyle ou phényl.

6. Emulsion aux halogénures d'argent conforme à la revendication 4, caractérisé en ce que les groupements $R^2$ et $R^3$, considérés ensemble, complètent un groupement hétéocylique qui contient un hétéroatome supplémentaire choisi dans le groupe constitué par les atomes d'azote, d'oxygène, de soufre et de sélénium.

7. Emulsion aux halogénures d'argent conforme à la revendication 4, caractérisée en ce que les groupements $R^2$ et $R^3$, considérés ensemble, complètent un groupement morpholino, pyrrolidyle, pyrrolyle ou pipéridine.

8. Emulsion aux halogénures d'argent conforme à la revendication 4, caractérisée en ce que l'agent de nucléation est choisi dans le groupe des composés

1-[4-(2-formylhydrazino)phényl]-3,3-diméthylthiourée,
1-[4-(2-formylhydrazino)phényl]-3,3-dibenzylthiourée,
1-[4-(2-acétylhydrazino)phényl]-3,3-dibenzylthiourée,
1-[4-(2-formylhydrazino)phényl]-3-méthyl-3-phénylthiourée,
4-[4-(2-formylhydrazino)phénylthiocarbamoyl]morpholine,
1-[4-(2-formylhydrazino)phénylthiocarbamoyl]-3-pyrroline,
1-[4-(2-formylhydrazino)phénylthiocarbamoyl]pyrrolidine et
1-[4-(2-formylhydrazino)phénylthiocarbamoyl]pipéridine.

9. Emulsion aux halogénures d'argent conforme à l'une des revendications 1 à 8, caractérisée en ce que l'émulsion contient un inhibiteur de voile.

10. Emulsion aux halogénures d'argent conforme à la revendication 9, caractérisée en ce que l'inhibiteur de voile est un benzotriazole utilisé à une concentration de 5 mg à 500 mg par mole d'halogénures d'argent.

11. Produit photographique qui comprend un support et une couche, caractérisé en ce qu'il comprend une couche d'une émulsion aux halogénures d'argent conforme à l'une des revendications 1 à 10.

12. Produit photographique conforme à la revendication 11, caractérisé en ce que cette couche comprend, en association, un composé formateur d'image de colorant.

13. Produit photographique conforme à la revendication 12, caractérisé en ce que ce composé formateur d'image de colorant est un composé qui libère un colorant par une réaction d'oxydoréduction, ce colorant présentant une couleur complémentaire de celle à laquelle les halogénures d'argent sont sensibles.

14. Produit photographique conforme à la revendication 13, caractérisé en ce qu'il comprend trois couches respectivement sensibles à la lumière bleue, verte et rouge.

15. Produit photographique, composite, pour diffusion-transfert, caractérisé en ce qu'il comprend un produit photographique conforme à l'une des revendications 11 à 14, une couche réceptrice d'image de colorant appliquée sur le support du produit photographique ou sur un support distinct, un réservoir contenant une solution de traitement basique et un développateur des halogénures d'argent, superficiel.

16. Procédé directement positif de formation d'une image visible dans un produit photographique exposé suivant une image caractérisé en ce qu'on traite le produit photographique conforme à l'une des revendications 11 à 15 par une composition de traitement aqueuse basique, en présence d'un développateur des halogénures d'argent, superficiel.

17. Procédé directement positif de formation d'une image conforme à la revendication 16,

caractérisé en ce que la composition de traitement basique présente un pH compris entre 13,9 et 11,8.

18. Procédé directement positif de formation d'image conforme à la revendication 16 ou 17, caractérisé en ce que la composition de traitement basique contient un inhibiteur de voile.

19. Procédé directement positif de formation d'image conforme à la revendication 18, caractérisé en ce que la composition de traitement aqueuse basique contient un inhibibiteur de voile de la classe du benzotriazole à une concentration comprise entre 500 mg/l et 10 g/l.

**Patentansprüche**

1. Silberhalogenidemulsion mit Silberhalogenidkörnern, die ein latentes Innenbild zu liefern vermögen und die an ihren Oberflächen einen Acylhydrazinophenylthioharnstoff-Keimbildner adsorbiert enthalten, dadurch gekennzeichnet, daß der Keimbildner der folgenden Formel entspricht:

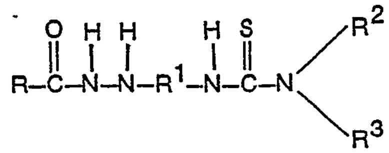

worin:

R ein Wasserstoffatom oder eine Cycloalkylgruppe, die durch Halogen-, Cyano- oder Alkoxygruppen substituiert sein kann, oder eine Alkylgruppe, die durch Halogen-, Alkoxy- oder Phenylgruppen substituiert sein kann,

$R^1$ eine Phenylengruppe oder eine Phenylengruppe, die durch Alkyl, Halogen oder Alkoxy substituiert ist und

$R^2$ und $R^3$ unabhängig voneinander

a) eine Alkyl-, Haloalkyl-, Alkoxyalkyl- oder Phenylalkyl- gruppe mit 1 bis 18 Kohlenstoffatomen,

b) eine Cycloalkylgruppe, die durch Halogen-, Cyano- oder Alkoxygruppen substituiert sein kann,

c) eine Phenylgruppe mit einer von einem positiven Hammett-Sigma-Wert abgeleiteten Elektronen abziehenden Charakteristik von weniger als +0,50 oder

d) eine Naphthylgruppe bedeuten, oder

$R^2$ und $R^3$ gemeinsam eine heterocyclische Gruppe mit einem 5- oder 6-gliedrigen Ring verfollständigen, der mindestens eine zusätzliches Heteroatom aux Stickstoff, Sauerstoff, Schwefel oder Selen aufweisen kann, wobei gilt, daß die Alkylgruppen, sofern nichts anderes angegeben ist, 1 bis 6 Kohlenstoffatome aufweisen und die Cycloalkylgruppen 3 bis 10 Kohlenstoffatome.

2. Silberhalogenidemulsion nach Anspruch 1, dadurch gekennzeichnet, daß sie 0,1 bis 500 mg des Keimbildners pro Mol Silberhalogenid enthält.

3. Silberhalogenidemulsion nach Anspruch 2, dadurch gekennzeichnet, daß sie 1 bis 100 mg des Keimbildners pro Mol Silberhalogenid enthält.

4. Silberhalogenidemulsion nach Anspruch 1, 2 oder 3, dadurch gekennzeichnet, daß der Keimbildner der folgenden Formel entspricht:

(II) 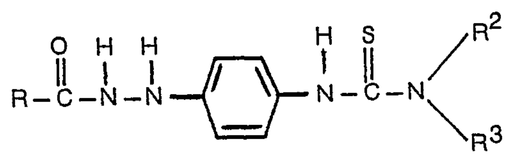

worin:

R ein Wasserstoffatom oder eine Methylgruppe und

$R^2$ und $R^3$ unabhängig voneinander eine Alkylgruppe mit 1 bis 6 Kohlenstoffatomen, eine Phenylalkylgruppe, in der der Alkylrest 1 bis 6 Kohlenstoffatome aufweist, oder eine Phenylgruppe mit einer von einem positiven Hammett-Sigma-Wert abgeleiteten Elektronen abziehenden Charakteristik von weniger als +0,50 bedeuten, oder

$R^2$ und $R^3$ gemeinsam einen gesättigten heterocyclischen Kern mit einem 5- oder 6-gliedrigen Ring vervollständigen, der als zusätzliches Heteroatom mindestens ein Stickstoff-, Sauerstoff-, Schwefel- oder Selenatom aufweist.

5. Silberhalogenidemulsion nach Anspruch 4, dadurch gekennzeichnet, daß $R^2$ und $R^3$ jeweils aus Methyl, Benzyl oder Phenyl bestehen.

6. Silberhalogenidemulsion nach Anspruch 4, dadurch gekennzeichnet, daß $R^2$ und $R^3$ gemeinsam einen heterocyclischen Ring vervollständigen, der ein zusätzliches Heteroatom aus N, O, S oder Se aufweist.

7. Silberhalogenidemulsion nach Anspruch 4, dadurch gekennzeichnet, daß $R^2$ und $R^3$ einen Morpholino-, einen Pyrrolidyl-, Pyrrolyl- oder Piperidinoring vervollständigen.

8. Silberhalogenidemulsion nach Anspruch 4, dadurch gekennzeichnet, daß der Keimbildner

besteht aus;

1-[4-(2-Formylhydrazino)phenyl]-3,3-dimethylthioharnstoff,
1-[4-(2-Formylhydrazino)phenyl]-3,3-dibenzylthioharnstoff,
1-[4-(2-Acetylhydrazino)phenyl]-3,3-dibenzylthioharnstoff,
1-[4-(2-Formylhydrazino)phenyl]-3-methyl-3-phenylthioharnstoff,
4-[4-(2-Formylhydrazino)phenylthiocarbamoyl]morpholin,
1-[4-(2-Formylhydrazino)phenylthiocarbamoyl]-3-pyrrolin,
1-[4-(2-Formylhydrazino)phenylthiocarbamoyl]pyrrolidin oder
1-[4-(2-Forymhydrazino)phenylthiocarbamoyl]piperidin.

9. Silberhalogenidemulsion nach einem der Ansprüche 1 bis 8, dadurch gekennzeichnet, daß die Emulsion ein Antischleiermittel enthält.

10. Silberhalogenidemulsion nach Anspruch 9, dadurch gekennzeichnet, daß das Antischleiermittel ein Benzotriazol in einer Konzentration von 5 bis 500 mg pro Mol Silberhalogenid ist.

11. Photographisches Element mit einem Träger und einer hierauf aufgetragenen Silberhalogenidemulsionsschicht, dadurch gekennzeichnet, daß das Element eine Silberhalogenidemulsion nach einem der Ansprüche 1 bis 10 aufweist.

12. Photographisches Element nach Anspruch 11, dadurch gekennzeichnet, daß der Schicht eine ein Farbstoffbild liefernde Verbindung zugeordnet ist.

13. Photographisches Element nach Anspruch 12, dadurch gekennzeichnet, daß die Verbindung aus einer einen Farbstoff freisetzenden Redox-Verbindung besteht, wobei der Farbstoff einen Farbton aufweist, der komplementär ist dem Farbton, demgegenüber das Silberhalogenid empfindlich ist.

14. Photographisches Element nach Anspruch 13, dadurch gekennzeichnet, daß es drei Schichten aufweist, die blau-, grün- bzw. rot-empfindlich sind.

15. Bildübertragungs-Filmeinheit, dadurch gekennzeichnet, daß sie ein photographisches Element nach einem der Ansprüche 11 bis 14 aufweist, eine Farbstoffbild-Empfangsschicht auf dem Träger oder einem anderen Träger, einen Behälter für eine alkalische Entwicklungsflüssigkeit und eine Oberflächen-Silberhalogenidentwicklerverbindung.

16. Direkt-positives Verfahren zur Herstellung eines sichtbaren Bildes in einem bildmäßig belichteten photographischen Element, dadurch gekennzeichnet, daß man ein Element nach einem der Ansprüche 11 bis 15 in einer wäßrigen alkalischen Entwicklungsflüssigkeit in Gegenwart einer Oberflächen-Entwicklerverbindung entwickelt.

17. Direkt-positives Verfahren nach Anspruch 16, dadurch gekennzeichnet, daß die wäßrige Entwicklungsflüssigkeit einen pH-Wert von 13,9 bis 11,8 aufweist.

18. Direkt-positives Verfahren nach Ansprüchen 16 oder 17, dadurch gekennzeichnet, daß die wäßrige alkalische Entwicklungsflüssigkeit ein Antischleiermittel enthält.

19. Direkt-positives Verfahren nach Anspruch 18, dadurch gekennzeichnet, daß die wäßrige alkalische Entwicklungsflüssigkeit ein Benzotriazol-Antischleiermittel in einer Konzentration von 500 mg bis 10 g pro Liter enthält.